(19) Europäisches Patentamt
European Patent Office
Office européen des brevets

(11) Veröffentlichungsnummer: **0 261 416**
**A2**

(12) # EUROPÄISCHE PATENTANMELDUNG

(21) Anmeldenummer: **87112233.9**

(22) Anmeldetag: **24.08.87**

(51) Int. Cl.4: **G01N 33/53**, G01N 33/531, G01N 33/535

(30) Priorität: **24.09.86 DE 3632343**

(43) Veröffentlichungstag der Anmeldung:
**30.03.88 Patentblatt 88/13**

(84) Benannte Vertragsstaaten:
**CH DE FR GB LI NL SE**

(71) Anmelder: **Battelle-Institut e.V.**
**Am Römerhof 35 Postfach 900 160**
**D-6000 Frankfurt/Main 90(DE)**

(72) Erfinder: **Dreher, Ralf, Dr.**
**Oberer Dorfgraben 61**
**D-6500 Mainz(DE)**
Erfinder: **Knoell, Hans-Emil, Dr.**
**Hirschberger Strasse 6**
**D-6905 Schriesheim(DE)**
Erfinder: **Frevert, Jürgen, Dr.**
**Heinestrasse 6**
**D-6000 Frankfurt am Main(DE)**
Erfinder: **Sailer, Johann, Dr.**
**Waldwinkel 9**
**D-5308 Rheinbach(DE)**

(74) Vertreter: **Sartorius, Peter, Dipl.-Ing.**
**Battelle-Institut e.V. Abteilung Patente Am Römerhof 35**
**D-6000 Frankfurt am Main 90(DE)**

(54) **Verfahren zum Nachweis von 1,2,2-Trimethylpropylmethylphosphofluoridad.**

(57) Zum Nachweis von 1,2,2-Trimethylpropyl-methylphosphofluoridad wird die nachzuweisende Verbindung oder ein Derivat davon an Tetanustoxoid kovalent gebunden. Mit Hilfe des erhaltenen Protein-Konjugats werden polyklonale oder monoklonale Antikörper, vorzugsweise in Kaninchen erzeugt. Der Nachweis wird anschließend in an sich bekannter Weise durch einen Enzymimmunoassay nach vorheriger Derivatisierung mit Brenzkatechin oder Hydroxylamin durchgeführt.

EP 0 261 416 A2

## Verfahren zum Nachweis von 1,2,2-Trimethylpropyl-methylphosphofluoridad

Die Erfindung betrifft ein Verfahren zum Nachweis von 1,2,2-Trimethylpropyl-methylphosphofluoridad, bei dem die nachzuweisende Verbindung oder ein Derivat davon chemisch an ein Protein kovalent gekoppelt wird und mit Hilfe des erhaltenen Protein-Konjugates Antikörper erzeugt werden und anschließend der Nachweis durch einen Enzymimmunoassay durchgeführt wird.

Organophosphorsäureester wie 1,2,2-Trimethylpropyl-methylphosphofluoridad, sind äußerst wirkungsvolle Inhibitoren der Acetylcholinesterase. Die Inhibition dieses Enzyms bedeutet meist den Tod des betroffenen Organismus. Zur raschen und eindeutigen Identifizierung dieser Verbindung sollten schnelle und sensitive Nachweisverfahren einsetzbar sein. Bisher wurden generell physikalisch-chemische Methoden, wie Gaschromatographie, Massenspektrometrie, dazu verwendet.

Diese Methoden erfordern jedoch eine zeit-und kostenaufwendige Geräteausrüstung, und die langwierigen Arbeiten setzen den Einsatz von hochspezialisiertem Personal voraus.

Seit der Entwicklung von Immunoassays steht ein empfindliches, aber einfach zu handhabendes Instrumentarium zur Verfügung, das es ermöglicht, niedermolekulare, biologisch relevante Moleküle wie z. B. Hormone, Arzneistoffe, Insektizide usw. in äußerst geringen Konzentrationen zu bestimmen. Grundsätzlich besteht hierbei die Schwierigkeit, daß niedermolekulare Verbindungen per se nicht immunogen sind, sie müssen erst mit hochmolekularen Proteinen verknüpft werden, um eine Immunantwort zu induzieren. Diese Proteinbindung ist der schwierigste Teil der Entwicklung, da nicht immer klar ist, wie das kleine Molekül mit dem Protein verknüpft werden muß, um die entscheidenden antigenen Eigenschaften zu exponieren. Weitere Schwierigkeiten sind die Verfügbarkeit funktioneller Gruppen am Molekül überhaupt, die eine chemische Derivatisierung, d. h. "Aktivierung" erlauben, und die Wahl eines geeigneten Spacers, der das Molekül räumlich etwas auf Abstand zum Protein hält, damit es dort nicht in der Tertiär-oder Quartär-Struktur vergraben und für eine antigene Erkennung unzugänglich bleibt.

Es sind in den letzten Jahren eine Reihe von chemischen Methoden entwickelt worden, um niedermolekulare Verbindungen mit Proteinen zu konjugieren, z. B. mit Carbodiimiden, Chlorformiaten, Diazolium-Salzen, wobei eine CO-NH-Bindung geknüpft wird, oder mit Diisocyanaten, Diiminoestern, Dihalonitrobenzol und Glutaraldehyd, die zu einer NH-R-HN-Bindung führen.

Die mit dem Protein-Konjugat gewonnenen Antikörper lassen sich dann in den verschiedenen immunologischen Testsystemem wie RIA und ELISA einsetzen.

Es ist bekannt, daß 4-Aminophenyl-1,2,2-Trimethylpropyl-methylphosphonat zur Antikörperherstellung zu verwenden ist (FEBS letters, 149, 147-151, 1982). In der genannten Veröffentlichung werden monoklonale Antikörper erzeugt. Zur Detektion von freiem 1,2,2-Trimethylpropyl-methylphosphofluoridad sind drei Reaktionsschritte erforderlich. Die Nachteile dieses Verfahrens liegen vor allem im Einsatz eines wenig effektiven Protein-Konjugates und an dem hohen Aufwand zur Isolierung der monoklonalen Antikörper. Dabei wird die Sensivität nicht erhöht. Ferner ist die Gesamtprozedur des bekannten Assays mit drei folgenden Reaktionsschritten langwierig und bedarf zusätzlicher und teurer Reagenzien.

Der vorliegenen Erfindung lag die Aufgabe zugrunde, einen Enzymimmunoassay zur sensitiven Erkennung von 1,2,2-Trimethylpropyl-methylphosphofluoridad zu entwickeln, der die Nachteile bekannter Assays nicht aufweist.

Diese Aufgabe ist erfindungsgemäß dadurch gelöst, daß ein Derivat der nachzuweisenden Verbindung an Tetanustoxoid Haemocyanin gekoppelt und mit dem entstehenden Protein-Konjugat polyklonale oder monoklonale, vorzugsweise polyklonale Antikörper erzeugt werden. Ansprüche 2 bis 5 betreffen bevorzugte Weiterbildungen des erfindungsgemäßen Verfahrens.

Das erfindungsgemäße Verfahren wird im nachfolgenden Beispiel näher erläutert.

Beispiel

1. Herstellung der polyklonalen Antikörper

Als Ausgangsverbindung wird das p-Aminophenyl-Derivat verwendet:

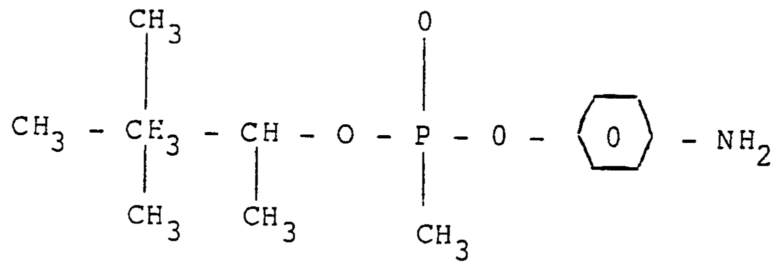

Dieses Derivat wird an succinyliertes Tetanustoxoid bzw. an Haemocyanin mit der gemischten Anhydrid-Methode gekoppelt.

Die präparierten Konjugate werden zur Immunisierung von Kaninchen und Meerschweinchen verwendet.

2. Immunoassay

Die gereinigte, hochspezifische Antikörperfraktion wird in an sich bekannter Weise zur Entwicklung eines kompetitiven Enzym-Immunoassay verwendet (Solid-phase tube Technik).

Zur Herstellung eines geeigneten Enzymkonjugats wird das p-Aminophenylderivat der nachzuweisenden Verbindung durch eine Perjodatoxidation an Peroxidase gekoppelt.

Die Reaktionsgefäße werden mit einer bestimmten Menge von Antikörper beschichtet. Die zu bestimmende Probe Brenzkatechin und die mit Peroxidase markierte Verbindung werden hinzupipettiert. Die nachzuweisende Verbindung aus der Probe und markierte Verbindung besetzen in einer konkurrierenden Reaktion die Antikörper an der Gefäßwand. Der Überschuß wird abgetrennt, störende Bestandteile ausgewaschen. Anschließend wird die Aktivität des an der Gefäßwand über den Antigenkörper-Komplex gebundenen Enzyms bestimmt.

Da der nachzuweisende Phosphorsäureester 1,2,2 Trimethylpropyl-methylphosphofluoridat aufgrund des fehlenden Fluor-Atoms im Antigen vom Testsystem nicht erkannt wurde, musste die nachzuweisende Verbindung vor der Bestimmung durch Umsetzung und Hydrolyse mit Brenzkatechin oder Hydroxylamin derivatisiert werden. Mit diesem Nachweissystem kann 1,2,2-Trimethylpropyl-methylphosphofluoridat in einem wässrigen System bis zu einer Konzentration von 3ng/ml oder 3ppb detektiert werden.

**Ansprüche**

1. Verfahren zum Nachweis von 1,2,2-Trimethylpropyl-methylphosphofluoridad, bei dem ein Derivat der nachzuweisenden Verbindung chemisch an ein Protein kovalent gekoppelt wird und mit Hilfe des erhaltenen Protein-Konjugates Antikörper erzeugt werden und anschließend der Nachweis durch einen Enzymimmunoassay durchgeführt wird, dadurch gekennzeichnet, daß ein Derivat der nachzuweisenden Verbindung an Tetanustoxoid oder Haemocyanin gekoppelt und mit dem entstehenden Protein-Konjugat polyklonale oder monoklonale Antikörper erzeugt werden

2. Verfahren nach Anspruch 1, dadurch gekennzeichnet, daß die nachzuweisende Verbindung zu einem Aminophenyl-, Aminoalkyl-, Carboxyalkyl-oder Epoxy-Derivat umgesetzt wird.

3. Verfahren nach Anspruch 1 oder 2, dadurch gekennzeichnet, daß polyklonale Antikörper im Kaninchen und Meerschweinchen erzeugt werden.

4. Verfahren nach einem der Ansprüche 1 bis 3, dadurch gekennzeichnet , daß die nachzuweisende Verbindung vor ihrem Nachweis im Enzymimmunoassay mittels Brenzkatechin oder Hydroxylamin derivatisiert wird.

5. Verfahren nach einem der Ansprüche 1 bis 4, dadurch gekennzeichnet, daß als Enzymkcnjugat im Immunoassay eine Verbindung zwischen dem p-Aminophenylderivat der nachzuweisenden Verbindung und Peroxidase verwendet wird, die mittels Perjodatoxidation hergestellt wird.